# EUROPEAN PATENT SPECIFICATION

(11) **EP 1 958 929 B1**
(45) Date of publication and mention of the grant of the patent: **14.11.2012**
(21) Application number: 06834208.8
(22) Date of filing: 07.12.2006
(51) Int. Cl.: C08G 65/22, C08G 65/26, C07C 41/03

(54) **PROCESS FOR PRODUCING POLYGLYCERYL ETHER DERIVATIVE**
VERFAHREN ZUR HERSTELLUNG EINES POLYGLYCERYLETHERDERIVATS
PROCÉDÉ DE SYNTHÈSE D'UN DÉRIVÉ DE POLYGLYCÉRYLÉTHER

(30) Priority: 09.12.2005 JP 2005356312; 08.11.2006 JP 2006302668
(43) Date of publication of application: 20.08.2008
(73) Proprietor: Kao Corporation, Chuo-ku Tokyo 103-8210 (JP)
(72) Inventor: SAITO, Akira, Wakayama 640-8580 (JP)
(74) Representative: HOFFMANN EITLE
(86) International application number: PCT/JP2006/324453
(87) International publication number: WO 2007/066723

(56) References cited:
- JP-A- 02 042 037
- JP-A- 58 000 927
- JP-A- 58 198 429
- JP-A- 2004 043 336
- JP-A- 2005 272 750
- US-A- 5 844 115

## Description

### FIELD OF THE INVENTION

The present invention relates to a process for producing polyglyceryl ether derivatives.

### BACKGROUND OF THE INVENTION

Polyglyceryl ether derivatives are useful compounds that are usable, for examples, as solvents, emulsifiers, dispersants, cleaning agents, foaming agents, etc.
There is conventionally known the method of producing the polyglyceryl ether derivatives by reacting an alcohol with glycidol. In the conventional method, after reacting the alcohol with an alkali, glycidol is dropped into the obtained reaction product to allow the alcohol to react therewith.
However, the above conventional method using the alkali tends to have problems such as low conversion rate of the alcohol used in the reaction and large burden upon purification for removal of the unreacted alcohol.
On the other hand, as a method for the reaction using a catalyst composed of a simple metal salt of rare earth element such as lanthanum triflate and ytterbium triflate, there are conventionally known, for example, the method for producing aromatic ketones (refer to Patent Document 1), the method for producing sugar ethers (refer to Patent Document 2), etc.
However, there is conventionally unknown any method for producing polyglyceryl ether derivatives by reacting an alcohol with glycidol in the presence of the simple metal salt of rare earth element as a catalyst.

Patent Document 1: JP 10-298131A
Patent Document 2: JP 9-1.57287A

### SUMMARY OF THE INVENTION

The present invention relates to a process for producing polyglyceryl ether derivatives by reacting an alcohol with glycidol in the presence of a simple metal salt of rare earth element as a catalyst.

### DETAILED DESCRIPTION OF THE INVENTION

The present invention relates to a process for producing polyglyceryl ether derivatives by addition reaction between an alcohol and glycidol which is capable of realizing a highly-selective addition reaction between the raw materials and enhancing a conversion rate of the alcohol.
The present inventors have found that when using a simple metal salt of rare earth element as a catalyst, a highly-selective reaction between the alcohol and glycidol can be realized, and the conversion rate of the alcohol can be enhanced. The term "highly-selective addition reaction" used herein means such a reaction in which production of polyglycidol by the polymerization between glycidol molecules themselves is suppressed, and glycidol is selectively reacted with the alcohol to preferentially produce the aimed polyglyceryl ether derivatives.

In the process for producing polyglyceryl ether derivatives cording to the present invention, the alcohol and glycidol which are as defined below are reacted with each other in the presence of a simple metal salt of rare earth element as a catalyst to produce the polyglyceryl ether derivatives.
In the present invention, as the alcohol that is one of the raw materials, there is used a monoalcohol (a) containing one hydroxyl group in a molecule thereof, and a polyol (b) containing 2 to 6 hydroxyl groups in a molecule thereof.
As the monoalcohol (a), there is used a compound represented by the general formula (1):

R¹-(OA)ₙ-OH (1)

wherein R¹ is a hydrocarbon group having 1 to 36 carbon atoms; A is an alkanediyl group having 2 to 4 carbon atoms; and n is a number of from 0 to 100 on the average.
In the general formula (1), R¹ is preferably a saturated or unsaturated, linear, branched or cyclic hydrocarbon group having 1 to 36 carbon atoms. Examples of the hydrocarbon group include linear, branched or cyclic alkyl groups having 1 to 36 carbon atoms, preferably 4 to 24 carbon atoms and more preferably 8 to 18 carbon atoms; and linear, branched or cyclic alkenyl groups having 2 to 36 carbon atoms, preferably 4 to 24 carbon atoms and more preferably 8 to 18 carbon atoms.

Examples of the linear, branched or cyclic alkyl groups include methyl, ethyl, n-propyl, isopropyl, various butyl groups, various pentyl groups, various hexyl groups, various octyl groups, various decyl groups, various dodecyl groups, various tetradecyl groups, various hexadecyl groups, various octadecyl groups, various icosyl groups, various tetracosyl groups, various triacontyl groups, cyclopentyl, cyclohexyl and cyclooctyl.
Examples of the linear, branched or cyclic alkenyl groups include propenyl, allyl, 1-butenyl, isobutenyl, various hexenyl groups, various octenyl groups, various decenyl groups, various dodecenyl groups, oleyl, cyclopentenyl, cyclohexenyl and cyclooctenyl.

In the general formula (1), A is preferably a linear or branched alkanediyl group having 2 to 4 carbon atoms. Example of the alkanediyl group include an ethylene group, a trimethylene group, a propane- 1,2-diyl group, a tetramethylene group, a 1-methyltrimethylene group and a 2-methyltrimethylene group. Among these alkanediyl groups, preferred are an ethylene group, a trimethylene group and a propane-1,2-diyl group.
In addition, n is preferably a number of from 0 to 20 and more preferably from 0 to 6. When n is 2 or more, a plurality of the (OA) groups may be the same or different from each other.

Specific examples of the monoalcohol (a) represented by the general formula (1) include methyl alcohol, ethyl alcohol, propyl alcohol, isopropyl alcohol, butyl alcohol, sec-butyl alcohol, pentyl alcohol, isopentyl alcohol, hexyl alcohol, cyclohexyl alcohol, 2-ethylhexyl alcohol, octyl alcohol, decyl alcohol, lauryl alcohol, myristyl alcohol, palmityl alcohol, stearyl alcohol, isostearyl alcohol, oleyl alcohol, ethylene glycol monomethyl ether, ethylene glycol monoethyl ether, ethylene glycol monopropyl ether, ethylene glycol monobutyl ether, propylene glycol monomethyl ether, propylene glycol monoethyl ether, propylene glycol monopropyl ether, propylene glycol monobutyl ether, polyethylene glycol monomethyl ether, polyethylene glycol monoethyl ether, polyethylene glycol monopropyl ether, polyethylene glycol monobutyl ether, polypropylene glycol monomethyl ether, polypropylene glycol monoethyl ether, polypropylene glycol monopropyl ether and polypropylene glycol monobutyl ether.
These monoalcohols (a) may be used alone or in the form of a mixture of any optional two or more thereof. Among these monoalcohols (a), from the viewpoint of a good applicability of the resultant polyglyceryl ether derivatives, especially preferred are lauryl alcohol, 2-ethylhexyl alcohol and isostearyl alcohol.

On the other hand, specific examples of the polyol (b) having 2 to 6 hydroxyl groups include ethylene glycol, diethylene glycol, polyethylene glycol, propylene glycol, dipropylene glycol, polypropylene glycol, 1,4-butylene glycol, 1,6-hexylene glycol, 1,8-octylene glycol, 1,10-decylene glycol, neopentyl glycol, trimethylol ethane, trimethylol propane, glycerol, diglycerol, pentaerythritol and sorbitol. These polyols (b) may be used alone or in the form of a mixture of any optional two or more thereof.
Among these polyols (b), from the viewpoint of a good applicability of the resultant polyglyceryl ether derivatives, preferred are trimethylol propane and glycerol, and more preferred is glycerol.

In the present invention, as the catalyst, there is used the simple metal salt of rare earth element (hereinafter occasionally referred to merely as a "rare earth-based catalyst"). The "simple metal salt" used herein means such a metal salt in the form of a primary compound except for a composite salt and a complex salt.
The simple metal salt of rare earth element may be usually used in the form of an inorganic acid salt and/or an organic acid salt. From the viewpoints of realizing the highly-selective addition reaction and enhancing a conversion rate of the alcohol, the inorganic acid salt is a perchloric acid salt, and the organic acid salt is a sulfonic acid salt.
Examples of the preferred rare earth element contained in the simple metal salt include lanthanoid elements such as scandium, yttrium, lanthanum, cerium, praseodymium, neodymium, samarium, europium, gadolinium, terbium, dysprosium, holmium, erbium, thulium, ytterbium and lutetium. Among these rare earth elements, more preferred are scandium, lanthanum, samarium, europium, erbium, lutetium and ytterbium, still more preferred are scandium, lanthanum, samarium and ytterbium, and further still more preferred are lanthanum and/or samarium.

Examples of the sulfonic acid salts of the rare earth elements are those compounds represented by the general formula (2):

M(OSO₂R²)ₓ (2)

wherein M is a rare earth element; R² is a hydrocarbon group, an alkoxyl group or a substituted or unsubstituted aryl group whose hydrogen atoms may be partially or wholly substituted with a fluorine atom; and x is an integer equal to a valence of M.

In the general formula (2), examples of the hydrocarbon group and the alkoxyl group as R² include those hydrocarbon and alkoxyl groups having 1 to 12 carbon atoms. Specific examples of the hydrocarbon group and the alkoxyl group as R² include methyl, ethyl, butyl, hexyl, octyl, decyl, dodecyl, methoxy, ethoxy, butoxy, hexyloxy, octyloxy, decyloxy, dodecyloxy, trifluoromethyl, pentafluoroethyl, nonafluorobutyl, trifluoromethoxy, pentafluoroethoxy and nonafluorobutoxy. Among these hydroxyl groups and alkoxyl groups, especially preferred is trifluoromethyl.
Examples of the substituted or unsubstituted aryl group as R² include those aryl groups having 6 to 25 carbon atoms in total. Specific examples of the aryl group as R² include phenyl, tolyl, xylyl, ethylphenyl, butylphenyl, octylphenyl, dodecylphenyl, naphthyl, methylnaphthyl and dimethylnaphthyl. Among these aryl groups, especially preferred are dodecylphenyl and tolyl.

Examples of the preferred sulfonic acid salts of the rare earth elements represented by the general formula (2) include triflates (trifluoromethanesulfonates), dodecylbenzenesulfonates and toluenesulfonates of scandium, lanthanum, samarium and ytterbium. Among these sulfonic acid salts, more preferred are triflates, dodecylbenzenesulfonates and toluenesulfonates of lanthanum and samarium.
In the present invention, the above simple metal salts of the rare earth elements may be used, as the catalyst, alone or in combination of any two or more thereof.

In the present invention, the alcohol and glycidol are reacted with each other in the presence of the rare earth-based catalyst to thereby produce the polyglyceryl ether derivative as aimed. When the alcohol is the monoalcohol represented by the general formula (1), as shown in the following reaction formula, the monoalcohol is reacted with the glycidol represented by the formula (3), thereby producing the polyglyceryl ether derivative represented by the general formula (4).

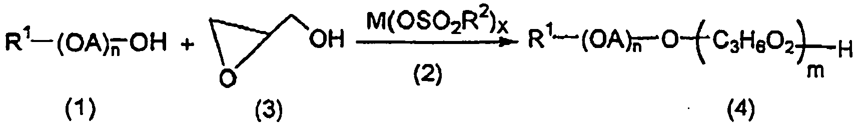

wherein m represents an average condensation degree; and R¹, R², A, M, n and x are the same as defined above.

In the general formula (4), (C₈H₆O₂) represents a polyglyceryl unit.
The polyglyceryl unit may have one or more structures selected from the group consisting of those structures represented by the following formulae.

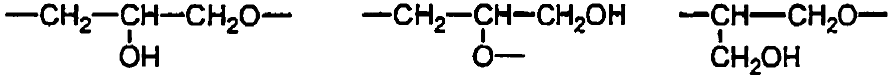

Further, the above structures may contain, as a part thereof, one or more structures selected from the group consisting of those structures represented by the following formulae.

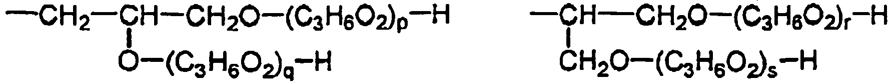

wherein p, q, r and s are respectively an integer of 1 or more; and (C₃H₆O₂) is the same as defined above.

The amounts of the monoalcohol (a) represented by the general formula (1) and the glycidol represented by the formula (3) which are used in the above reaction may be appropriately determined according to a desired average condensation degree m of the obtained polyglyceryl ether derivative represented by the general formula (4). The average condensation degree m used herein is defined by the value that is obtained by dividing the molar number obtained by subtracting moles of glycidol consumed by polymerization between glycidol molecules from moles of glycidol used in the reaction, by the molar number of the alcohol used in the reaction. The average condensation degree m is usually from 1 to 20 and preferably from 1 to 12. Therefore, the glycidol represented by the formula (3) is used in an amount of usually from 0.1 to 40 mol, preferably from 0.5 to 24 mol, more preferably from 1 to 20 mol and still more preferably from 1 to 12 mol per 1 mol of the monoalcohol (a) represented by the general formula (1).
Also, when the alcohol is the polyol (b) having 2 to 6 hydroxyl groups, the amounts of the polyol (b) and the glycidol which are used in the above reaction may be appropriately determined according to a desired amount of the polyglyceryl groups added to the plural hydroxyl groups of the polyol (b) and a desired average condensation degree of the polyglyceryl groups.
Meanwhile, when glycerol is used as the polyol, a polyglycerol represented by the general formula (5) is produced as shown in the following reaction formula.

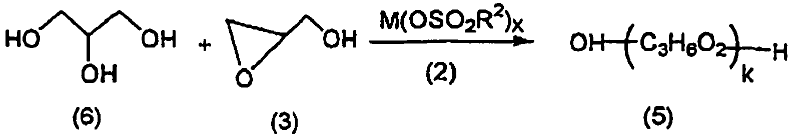

wherein k represents an average condensation degree; and M, R², x and (C₈H₆O₂) are the same as defined above.

The amounts of the glycerol represented by the formula (6) and the glycidol represented by the formula (3) which are used in the above reaction may be appropriately determined according to a desired average condensation degree k of the obtained polyglycerol represented by the general formula (5). The average condensation degree k is usually from 1 to 20 and preferably from 2 to 10. Therefor, the glycidol represented by the formula (3) is used in an amount of usually from 1 to 20 mol and preferably from 2 to 10 mol per 1 mol of the glycerol represented by the formula (6).

The amount of the rare earth-based catalyst used in the present invention is usually from 0.001 to 0.2 mol, preferably 0.002 to 0.1 mol and more preferably from 0.005 to 0.05 mol per 1 mol of the alcohol used, from the viewpoint of a good balance between reaction rate and economy.
Although the reaction may be conducted under a solvent-free condition, an organic solvent may be used in an appropriate amount for the purpose of facilitating mixing of the raw materials. Examples of the organic solvent include hexane, diethyl ether, tetrahydrofuran, dichloromethane, acetonitrile, nitromethane, benzene, toluene, xylene, chloroform, cyclohexane, dimethyl sulfoxide, dimethylformamide and dimethylacetamide. In addition, the reaction may be conducted in air, but is preferably conducted in an inert gas, for example, in a nitrogen atmosphere or in an argon atmosphere, for the purpose of suppressing production of by-products.

The reaction temperature varies depending upon kind of the alcohol used, kind and amount of the catalyst used, etc. From the viewpoints of practical reaction time, yield and quality of the resultant product, the reaction temperature is usually from about 0 to about 200°C, preferably from 30 to 170°C, more preferably from 50 to 150°C and still more preferably from 80 to 130°C. Also, the reaction time varies depending upon the reaction conditions, and is, therefore, not particularly limited. The reaction time is usually from about 30 min to about 100 h, preferably from 1 to 50 h and more preferably from 1 to 30 h.
After completion of the reaction, the obtained reaction solution is optionally subjected to washing treatment according to the requirements, and then treated by the methods such as filtration, distillation and extraction, thereby obtaining the polyglyceryl ether derivative as aimed. If required, the resultant polyglyceryl ether derivative may be further purified by an ordinary method such as silica gel column chromatography, distillation and recrystallization. At this time, the used rare earth-based catalyst may be recovered and reused. For this purpose, the polyglyceryl ether derivative is preferably obtained by the extraction. More specifically, after extracting the polyglyceryl ether derivative, an aqueous solution containing the rare earth-based catalyst is recovered, and water is distilled off from the aqueous solution, thereby isolating the rare earth-based catalyst therefrom. Further, if required, the thus isolated rare earth-based catalyst may be purified and then reused in the process of the present invention.

### EXAMPLES

### EXAMPLE 1

A 300 mL four-necked flask was charged with 95.1. g (0.50 mol) of lauryl alcohol and 2.94 g (0.0050 mol) of lanthanum triflate, and the contents of the flask were heated to 90°C while stirring under a nitrogen flow. Next, while maintaining the temperature of 90°C, 111.12 g (1.5 mol) of glycidol was dropped into the flask over 24 h, and the contents of the flask were continuously stirred for 2 h, thereby obtaining 209.1 g of a reaction product. As a result of analyzing the thus obtained reaction product by gas chromatography, it was confirmed that lauryl polyglycerol ether was produced (conversion rate of glycidol: 99.9% or more; in the following examples and comparative example, it was confirmed in the same manner). After completion of the reaction, the amount of unreacted lauryl alcohol remaining in the reaction solution was determined by gas chromatography. As a result, it was confirmed that the residual amount of the unreacted lauryl alcohol was 5.9% by mass (conversion rate of the alcohol: 87%), and the average condensation degree of the obtained lauryl polyglyceryl ether was 3.3. Meanwhile, the residual amount of the added glycidol in the reaction solution was less than 0.1% by mass, and the content of polyglycerol contained in the reaction product was 2.2% by mass.

### COMPARATIVE EXAMPLE 1

A 300 mL four-necked flask was charged with 57.0 g (0.30 mol) of lauryl alcohol and 4.42 g (0.060 mol) of potassium methylate, and the contents of the flask were heated to 95°C while stirring under a reduced pressure of 25 kPa to distil off methanol therefrom. Next, 66.7 g (0.90 mol) of glycidol was dropped into the flask at 95°C under a nitrogen flow over 24 h, and the contents of the flask were continuously stirred for 2 h. After completion of the reaction, 3.10 g (0.030 mol) of sulfuric acid and 10 g of water were added to the reaction solution to neutralize the catalyst, thereby obtaining 126.6 g of a reaction product (conversion rate of glycidol: 99.9% or more). After completion of the reaction, the amount of unreacted lauryl alcohol remaining in the reaction solution was determined by gas chromatography. As a result, it was confirmed that the residual amount of the unreacted lauryl alcohol was 20.2% by mass (conversion rate of the alcohol: 55%), and the average condensation degree of the obtained lauryl polyglyceryl ether was 4.4. Meanwhile, the residual amount of the added glycidol in the reaction solution was less than 0.1% by mass, and the content of polyglycerol in the reaction product was 10% by mass.

### EXAMPLE 2

A 300 mL four-necked flask was charged with 46,1 g (0.50 mol) of glycerol and 2.94 g (0.0050 mol) of lanthanum triflate, and the contents of the flask were heated to 120°C under a nitrogen flow. Next, while maintaining the temperature of 120°C, 111.18 g (1.5 mol) of glycidol was dropped into the flask over 10 h, and the contents of the flask were continuously stirred for 2 h, thereby obtaining 160.2 g of a reaction product (conversion rate of glycidol: 99.9% or more). After completion of the reaction, the amount of unreacted glycerol remaining in the reaction solution was determined by gas chromatography. As a result, it was confirmed that the residual amount of the unreacted glycerol was 3.5% by mass (conversion rate of glycerol: 88%). Meanwhile, the residual amount of the added glycidol in the reaction solution was less than 0.1% by mass.

### EXAMPLE 3

A 300 mL four-necked flask was charged with 95.1 g (0.50 mol) of lauryl alcohol and 5.58 g (0.0050 mol) of lanthanum tris(dodecylbenzenesulfonate), and the contents of the flask were heated to 120°C while stirring under a nitrogen flow. Next, while maintaining the temperature of 120°C, 111.12 g (1.50 mol) of glycidol was dropped into the flask over 10 h, and the contents of the flask were continuously stirred for 2 h, thereby obtaining 211.1 g of a reaction product (conversion rate of glycidol: 99.9% or more). After completion of the reaction, the amount of unreacted lauryl alcohol remaining in the reaction solution was determined by gas chromatography. As a result, it was confirmed that the residual amount of the unreacted alcohol was 4.1% by mass (conversion rate of the alcohol: 91%), and the average condensation degree of the obtained lauryl polyglyceryl ether was 3.1. Meanwhile, the residual amount of the added glycidol in the reaction solution was less than 0.1% by mass, and the content of polyglycerol in the reaction product was 3.9% by mass.

### EXAMPLE 4

A 300 mL four-necked flask was charged with 57.0 g (0.30 mol) of lauryl alcohol and 1.83 g (0.0030 mol) of samarium triflate, and the contents of the flask were heated to 120°C while stirring under a nitrogen flow. Next, while maintaining the temperature of 120°C, 66.67 g (0.90 mol) of glycidol was dropped into the flask over 10 h, and the contents of the flask were continuously stirred for 2 h, thereby obtaining 123.5 g of a reaction product (conversion rate of glycidol: 99.9% or more). After completion of the reaction, the amount of unreacted lauryl alcohol remaining in the reaction solution was determined by gas chromatography. As a result, it was confirmed that the residual amount of the unreacted lauryl alcohol was 8.8% by mass (conversion rate of the alcohol: 81%), and the average condensation degree of the obtained lauryl polyglyceryl ether was 3.5. Meanwhile, the residual amount of the added glycidol in the reaction solution was less than 0.1% by mass, and the content of polyglycerol in the reaction product was 3.0% by mass.

### EXAMPLE 5

A 300 mL four-necked flask was charged with 95.1 g (0.50 mol) of lauryl alcohol and 2.73 g (0.0050 mol) of lanthanum perchlorate hexahydrate, and the contents of the flask were heated to 90°C while stirring under a nitrogen flow. Next, while maintaining the temperature of 90°C, 111.12 g (1.5 mol) of glycidol was dropped into the flask over 6 h, and the contents of the flask were continuously stirred for 2 h, thereby obtaining 208.9 g of a reaction product (conversion rate of glycidol: 99.9% or more). After completion of the reaction, the amount of unreacted lauryl alcohol remaining in the reaction solution was determined by gas chromatography. As a result, it was confirmed that the residual amount of the unreacted lauryl alcohol was 9.6% by mass (conversion rate of the alcohol: 79%), and the average condensation degree of the obtained lauryl polyglyceryl ether was 3.3. Meanwhile, the residual amount of the added glycidol in the reaction solution was less than 0.1% by mass, and the content of polyglycerol in the reaction product was 6.7% by mass.

### EXAMPLE 6

A 300 mL four-necked flask was charged with 111.4 g (0.50 mol) of di(ethylene glycol) 2-ethylhexyl ether and 2.93 g (0.0050 mol) of lanthanum triflate, and the contents of the flask were heated to 90°C while stirring under a nitrogen flow. Next, while maintaining the temperature of 90°C, 111.12 g (1.5 mol) of glycidol was dropped into the flask over 6 h, and the contents of the flask were continuously stirred for 2 h, thereby obtaining 225.5 g of a reaction product (conversion rate of glycidol: 99.9% or more). After completion of the reaction, the amount of unreacted alcohol remaining in the reaction solution was determined by gas chromatography. As a result, it was confirmed that the residual amount of the unreacted alcohol was 13.3% by mass (conversion rate of the alcohol: 73%), and the average condensation degree of the obtained di(ethylene glycol) 2-ethylhexyl polyglyceryl ether was 3.5. Meanwhile, the residual amount of the added glycidol in the reaction solution was less than 0.1% by mass, and the content of polyglycerol in the reaction product was 7.8% by mass.

### INDUSTRIAL APPLICABILITY

In the process for producing the polyglyceryl ether derivatives according to the present invention, it is possible to realize a highly-selective addition reaction of an alcohol and glycidol, and enhance a conversion rate of the alcohol. In addition, the obtained polyglyceryl ether derivatives are useful, for example, as a solvent, an emulsifier, a dispersant, a cleaning agent, a foaming agent, etc.

## Claims

1. A process for producing a polyglyceryl ether derivative, comprising the step of reacting an alcohol with glycidol in the presence of a simple metal salt of rare earth element as a catalyst,
wherein the alcohol is a compound represented by the general formula (1):
R¹-(OA)ₙ-OH (1)
wherein R¹ is a hydrocarbon group having 1 to 36 carbon atoms; A is an alkanediyl group having 2 to 4 carbon atoms; and n is a number of from 0 to 100 on the average,
or wherein the alcohol is a polyol having 2 to 6 hydroxyl groups,
wherein the simple metal salt of rare earth elements is a perchloric acid salt or a salt of the formula (2):
M(OSO₂R²)ₓ
wherein M is a rare earth element, R² is a hydrocarbon group having 1 to 12 carbon atoms, an alkoxyl group having 1 to 12 carbon atoms or a substituted or unsubstituted aryl group having 6 to 25 carbon atoms in total and x is an integer equal to a valence of M; and wherein the rare earth element is selected from the lanthanoid elements.

2. The process according to claim 1, wherein the rare earth element is lanthanum and/or samarium.

3. The process according to claim 1 or 2, wherein the inorganic acid salt of the rare earth element is a perchloric acid salt thereof.

4. The process according to claim 1 or 2, wherein the organic acid salt of the rare earth element is a sulfonic acid salt thereof having the formula (2) as defined in claim 1.

5. The process according to any one of claims 1 to 4, wherein the simple metal salt of the rare earth element is used in an amount of from 0.001 to 0.2 mol per 1 mol of the alcohol.

## Patentansprüche

1. Verfahren zur Erzeugung eines
Polyglyceryletherderivates, umfassend den Schritt der Reaktion eines Alkohols mit Glycidol in der Gegenwart eines einfachen Metallsalzes eines Seltenerdelementes als Katalysator,
worin der Alkohol eine Verbindung ist, dargestellt durch die allgemeine Formel (1):
R¹-(OA)ₙ-OH (1)
worin R¹ eine Kohlenwasserstoffgruppe mit 1 bis 36 Kohlenstoffatomen ist, A eine Alkandiylgruppe mit 2 bis 4 Kohlenstoffatomen ist und n eine Zahl von 0 bis 100 im Schnitt ist,
oder worin der Alkohol ein Polyol mit 2 bis 6 Hydroxylgruppen ist,
worin das einfache Metallsalz der Seltenerdelemente ein Perchlorsäuresalz oder ein Salz der Formel (2) ist:
M(OSO₂R²)ₓ
worin M ein Seltenerdelement ist, R² eine Kohlenwasserstoffgruppe mit 1 bis 12 Kohlenstoffatomen, Alkoxygruppe mit 1 bis 12 Kohlenstoffatomen oder eine substituierte oder unsubstituierte Arylgruppe mit 6 bis 25 Kohlenstoffatomen insgesamt ist und x eine ganze Zahl ist, die einer Valenz von M gleich ist, und worin das Seltenerdelement ausgewählt ist aus Lanthanoidelementen.

2. Verfahren nach Anspruch 1, worin das Seltenerdelement Lanthan und/oder Samarium ist.

3. Verfahren nach Anspruch 1 oder 2, worin das anorganische Säuresalz des Seltenerdelementes ein Perchlorsäuresalz davon ist.

4. Verfahren nach Anspruch 1 oder 2, worin das organische Säuresalz des Seltenerdelementes ein Sulfonsäuresalz davon mit der Formel (2) wie in Anspruch 1 definiert ist.

5. Verfahren nach einem der Ansprüche 1 bis 4, worin das einfache Metallsalz des Seltenerdelementes in einer Menge von 0,001 bis 0,2 Mol pro 1 Mol des Alkohols verwendet wird.

## Revendications

1. Procédé de production d'un dérivé de polyglycéryléther, comprenant l'étape de faire réagir un alcool avec du glycidol en présence d'un sel métallique simple d'élément des terres rares en tant que catalyseur,
dans lequel l'alcool est un composé représenté par la formule générale (1) :
R¹-(OA)ₙ-OH (1)
où R¹ est un groupe hydrocarboné ayant 1 à 36 atomes de carbone ; A est un groupe alcanediyle ayant 2 à 4 atomes de carbone ; et n est un nombre allant de 0 à 100 en moyenne,
ou dans lequel l'alcool est un polyol ayant 2 à 6 groupes hydroxyles,
où le sel métallique simple d'éléments des terres rares est un sel de l'acide perchlorique ou un sel de la formule (2) :
M(OSO₂R²)ₓ
où M est un élément des terres rares, R² est un groupe hydrocarboné ayant 1 à 12 atomes de carbone, un groupe alcoxyle ayant 1 à 12 atomes de carbone ou un groupe aryle substitué ou non substitué ayant 6 à 25 atomes de carbone au total et x est un entier égal à une valence de M ; et où l'élément des terres rares est choisi parmi les éléments des lanthanides.

2. Procédé selon la revendication 1, dans lequel l'élément des terres rares est le lanthane et/ou le samarium.

3. Procédé selon la revendication 1 ou 2, dans lequel le sel d'acide inorganique de l'élément des terres rares est un sel d'acide perchlorique de celui-ci.

4. Procédé selon la revendication 1 ou 2, dans lequel le sel d'acide organique de l'élément des terres rares est un sel d'acide sulfonique de celui-ci ayant la formule (2) tel que définie dans la revendication 1.

5. Procédé selon l'une quelconque des revendications 1 à 4, dans lequel le sel métallique simple de l'élément des terres rares est utilisé en une quantité allant de 0,001 à 0,2 mole par 1 mole de l'alcool.
